# EUROPEAN PATENT APPLICATION

(11) **EP 2 290 367 A1**
(43) Date of publication of application: **02.03.2011**
(21) Application number: 10174487.8
(22) Date of filing: 30.08.2010
(51) Int. Cl.: G01N 33/558, G01N 33/58

(54) **Method for detecting objective substance and kit for detecting objective substance**

(30) Priority: 31.08.2009 JP 2009199284
(71) Applicant: SYSMEX CORPORATION, Kobe-shi, Hyogo 651-0073 (JP)
(72) Inventor: Hazama, Shunsuke, Kobe-shi, Hyogo 651-0073 (JP); Sakai, Yasuhiro, Kobe-shi, Hyogo 651-0073 (JP)
(74) Representative: Paemen, Liesbet R.J.

(57) **Abstract**

A method for detecting an objective substance in a sample using a chromatographic test device having a detection region on which a capture substance capable of binding to the objective substance is immobilized, comprising:mixing the sample and an enzyme-labeled substance capable of binding to the objective substance; trapping the objective substance binding to the enzyme-labeled substance by the capture substance in the detection region, by adding the liquid mixture to the chromatographic test device; adding an anionic surfactant-containing reagent to the detection region of the chromatographic test device in which the objective substance is captured; reacting a luminescent substrate with the enzyme of the enzyme-labeled substance binding to the capture substance through the objective substance in the detection region; and detecting the objective substance in the sample by detecting luminescence generated from the detection region by the reaction of the luminescent substrate with the enzyme. A kit is also disclosed.

## Description

### FIELD OF THE INVENTION

The invention relates to a method for detecting an objective substance and to a kit for detecting an objective substance.

### BACKGROUND

A method for detecting an objective substance in a test sample collected from an organism, such as blood, serum, a throat swab, a nasal swab, pituita, or urine is performed using chromatography. In this method, a capture substance capable of binding to the objective substance in the sample is first immobilized on the detection region of a chromatographic test device. A liquid mixture of the sample and a marker capable of binding to the objective substance is developed on the chromatographic test device, so that the objective substance labeled with the marker is captured in the detection region. At this time, a sandwich-like complex of marker-objective substance-capture substance is formed in the detection region. When colored latex particles or colloidal metal particles are used as a component of the marker, the detection region having the captured marker is colored. In the chromatography, the objective substance in the sample can be detected by observing the coloring of the detection region.

However, the chromatographic technique using colored latex particles or the like has low detection sensitivity and has difficulty in detecting an objective substance when the content of the objective substance in the sample is very low.

In recent years, therefore, chromatographic methods for detecting an objective substance with high sensitivity have been proposed in which a fluorescent or chemiluminescent substance is used as a marker component in place of colored latex particles.
For example, JP Laid-Open Pat. 2002-267671 discloses an immunochromatographic method using a fluorescent substance as a marker component. JP Laid-Open Pat. H8-5637 discloses an antibody detection method using acridinium, a chemiluminescent substance, as a marker component.
However, these methods cannot achieve the detection sensitivity required in the market.

### SUMMARY

The scope of the present invention is defined solely by the appended claims, and is not affected to any degree by the statements within this summary.
(1)A first aspect of the present invention is a method for detecting an objective substance in a sample using a chromatographic test device having a detection region on which a capture substance capable of binding to the objective substance is immobilized, comprising: mixing the sample and an enzyme-labeled substance capable of binding to the objective substance; trapping the objective substance binding to the enzyme-labeled substance by the capture substance in the detection region, by adding the liquid mixture to the chromatographic test device; adding an anionic surfactant-containing reagent to the detection region of the chromatographic test device in which the objective substance is captured; reacting a luminescent substrate with the enzyme of the enzyme-labeled substance binding to the capture substance through the objective substance in the detection region; and detecting the objective substance in the sample by detecting luminescence generated from the detection region by the reaction of the luminescent substrate with the enzyme. According to this method, the objective substance can be detected with high sensitivity in a chromatographic technique using enzymatic chemiluminescence.
(2) The method of(1), wherein the anionic surfactant is selected from sodium dodecyl sulfate, sodium dodecylbenzenesulfonate, sodium n-decyl sulfate, sodium lauryl sulfoacetate, sodium 1-dodecanesulfonate, p-octylbenzenesulfonic acid, 1-octanesulfonic acid, sodium 1-decanesulfonate, sulfuric acid sodium octane-1-yl, and sodium POE(2) lauryl ether sulfate.
(3) The method of (1) or (2), wherein the addition of the anionic surfactant-containing reagent to the detection region of the chromatographic test device is performed by developing the anionic surfactant-containing reagent on the chromatographic test device.
(4) The method of any one of (1) to (3), wherein the anionic surfactant-containing reagent further contains a buffer; and the anionic surfactant-containing reagent contains the anionic surfactant at a concentration of 0.0001 to 0.1% by weight. When such a reagent is used, the environment for the luminescence of the labeled material-objective substance-capture material can be adjusted, while the chromatographic test device is cleaned.
(5) The method of any one of (1) to (4), wherein the luminescent substrate is a dioxetane; and the enzyme of the enzyme-labeled substance is alkaline phosphatase.
(6) The method of any one of (1) to (5), wherein the capture substance is an antibody, and the enzyme-labeled substance is an enzyme-labeled antibody.
(7) The method of any one of (1) to (6), wherein the detection of the objective substance comprises quantifying the objective substance in the sample by measuring the intensity of the luminescence generated from the detection region.
(8) The method of any one of (1) to (7), wherein the chromatographic test device is a lateral flow type chromatographic test device or a flow through type chromatographic test device.
(9) The second aspect of the present invention is a chromatography test kit for detecting an objective substance in a sample, comprising: an enzyme-labeled substance capable of binding to the objective substance; a chromatographic test device having a detection region on which a capture substance capable of binding to the objective substance is immobilized; an anionic surfactant-containing reagent; and a luminescent substrate for the enzyme-labeled substance, the substrate being capable of acting on the enzyme-labeled substance and causing a luminescent reaction. This feature makes it possible to detect the objective substance with high sensitivity in a chromatographic technique using enzymatic chemiluminescence.
(10) The kit of (9), wherein the anionic surfactant is selected from sodium dodecyl sulfate, sodium dodecylbenzenesulfonate, sodium n-decyl sulfate, sodium lauryl sulfoacetate, sodium 1-dodecanesulfonate, p-octylbenzenesulfonic acid, 1-octanesulfonic acid, sodium 1-decanesulfonate, sulfuric acid sodium octane-1-yl, and sodium POE(2) lauryl ether sulfate.
(11) The kit of (9) or (10), wherein the anionic surfactant-containing reagent further contains a buffer; and the anionic surfactant-containing reagent contains the anionic surfactant at a concentration of 0.0001 to 0.1% by weight.
(12) The kit of any one of (9) to (11), wherein the luminescent substrate is a dioxetane.
(13) The kit of any one of (9) to (12), wherein the enzyme of the enzyme-labeled substance is alkaline phosphatase.
(14) The kit of any one of (9) to (13), wherein the capture substance is an antibody, and the enzyme-labeled substance is an enzyme-labeled antibody.
(15) The kit of any one of (9) to (14), wherein the chromatographic test device is a lateral flow type chromatographic test device or a flow through type chromatographic test device.

### BRIEF DESCRIPTION OF THE DRAWINGS

Fig. 1 shows a plan view (part (a)) and a side view (part (b)) of a chromatographic test device according to an embodiment of the invention;
Fig. 2 is a perspective view of a case 10 in which the chromatographic test device according to an embodiment of the invention is housed;
Fig. 3 shows a plan view (part (a)) and an X-X cross-sectional view (part (b)) of a chromatographic test device according to an embodiment of the invention;
Fig. 4 is a schematic diagram of reagent vessels according to an embodiment of the invention;
Fig. 5 is a photograph showing the result of the detection of luminescence in Example 1;
Fig. 6 is a photograph showing the result of the detection of luminescence in Comparative Example 1;
Fig. 7 is a photograph showing the result of the detection of luminescence in Example 2;
Fig. 8 is a photograph showing the result of the detection of luminescence in Example 3; and
Fig. 9 is a photograph showing the result of the detection of luminescence in Example 4.

### DETAILED DESCRIPTION OF EMBODIMENTS

The inventors have made the invention based on the finding that in a chromatographic technique using enzymatic chemiluminescence, the intensity of the luminescence from the luminescent substrate can be enhanced by the addition of an anionic surfactant-containing reagent to the detection region after the development of a liquid mixture of an objective substance and an enzyme-labeled substance.

In an embodiment of the invention, the method for detecting an objective substance is a method for detecting an objective substance in a sample using a chromatographic test device having a detection region on which a capture substance capable of binding to the objective substance is immobilized, which includes: mixing the sample and an enzyme-labeled substance capable of binding to the objective substance; adding the resulting liquid mixture to the chromatographic test device so that the capture substance in the detection region can capture the objective substance binding to the enzyme-labeled substance; adding an anionic surfactant-containing reagent to the detection region of the chromatographic test device in which the objective substance is captured; allowing a luminescent substrate to react with the enzyme of the enzyme-labeled substance binding to the capture substance through the objective substance in the detection region; and detecting luminescence generated from the detection region by the reaction of the luminescent substrate with the enzyme to detect the objective substance in the sample. As used herein, the term "detecting an objective substance" is intended to include not only determining the presence or absence of the objective substance in the sample but also quantifying the objective substance in the sample.

In the mixing step, the sample and the enzyme-labeled substance binding to the objective substance are mixed to form a liquid mixture. If the sample contains the objective substance, the objective substance will form a complex with the enzyme-labeled substance.

In an embodiment of the invention, any liquid sample potentially containing an objective substance may be used. In an embodiment of the invention, the sample used is preferably a biological sample. Examples of the sample include blood, serum, blood plasma, bile, gastrointestinal secretion, lymph fluid, bone marrow fluid, saliva, pituita, urine, tissue extracts, tissue homogenates, cell extracts, and cell homogenates.

The objective substance contained in the sample may be of any type. Examples of the objective substance include antigens, antibodies, hormones, hormone receptors, lectins, lectin-binding saccharides, drugs or drug metabolites, drug receptors, nucleic acids and fragments thereof, and so on. Examples also include cells such as bacteria, protista and fungi, viruses, proteins, and polysaccharides. Specific examples include viruses such as influenza viruses, parainfluenza viruses, RS viruses, Mycoplasma pneumoniae, rotaviruses, caliciviruses, coronaviruses, adenoviruses, enteroviruses, herpesviruses, human immunodeficiency viruses, and hepatitis viruses; cells such as Escherichia coli, Staphylococcus aureus, Streptococcus pneumoniae, Streptococcus pyogenes, Mycoplasma pneumoniae, and Plasmodium falciparum; pathogens of various diseases such as digestive diseases, central nervous system diseases and hemorrhagic fevers, and metabolites of pathogens; tumor markers such as carcinoembryonic antigens and CYFRA; and hormones.

The enzyme-labeled substance capable of forming a complex with the objective substance in the sample may be any substance that is capable of specifically binding to the objective substance and labeled with an enzyme. When the objective substance is an antigen or an antibody, an antibody or antigen capable of binding to the antigen or antibody by an antigen-antibody reaction may be used as the substance capable of specifically binding to the objective substance. Alternatively, a substance capable of binding to the objective substance based on a biological affinity such as ligand-receptor binding may also be used. The enzyme-labeled substance is preferably an antibody labeled with an enzyme (an enzyme-labeled antibody).

The enzyme of the enzyme-labeled substance may be any known enzyme generally used in the art. Examples of the enzyme include α-galactosidase, alkaline phosphatase, or peroxidase, and alkaline phosphatase is preferred.

Any known method may be used for labeling with the enzyme. For example, labeling with alkaline phosphatase may be performed by glutaraldehyde method, periodic acid crosslinking method, maleimide crosslinking method, carbodiimide method, activated ester method, or the like.

In the capturing step, the liquid mixture obtained in the mixing step is added to the chromatographic test device so that the capture substance in the detection region can capture the objective substance. The chromatographic test device used in this step has a detection region. A capture substance capable of capturing the objective substance in the sample is immobilized on the detection region. Therefore, the complex of the enzyme-labeled substance and the objective substance in the liquid mixture is captured by the capture substance in the detection region.

Examples of the chromatographic test device include a lateral flow type chromatographic test device and a flow through type chromatographic test device.
The lateral flow type chromatography refers to a method including adding a sample dropwise to a membrane having the detection region on which the capture substance is immobilized, and developing the sample parallel to the membrane so that the objective substance captured in the detection region can be detected. The flow through type chromatography refers to a method including adding a sample containing the objective substance dropwise to a membrane having a surface on which the capture substance for capturing the objective substance is immobilized, and allowing the sample to pass perpendicular to the membrane so that the objective substance captured on the surface of the membrane can be detected. In both methods, the captured objective substance is labeled with the enzyme-labeled substance. The enzyme of the enzyme-labeled substance is allowed to react with the luminescent substrate to generate luminescence as described below, so that the objective substance can be detected.

The capture substance immobilized on the detection region may be any substance capable of specifically biding to the objective substance in the sample. Examples of the specific binding between the objective substance and the capture substance include binding caused by an antigen-antibody reaction and binding based on a biological affinity such as ligand-receptor binding. When the objective substance is an antigen, an antibody to the antigen is preferably used for the enzyme-labeled substance or the capture substance. In particular, antibodies used for the enzyme-labeled substance and the capture substance preferably recognize different sites (epitopes) of the antigen, respectively. When the objective substance is an antibody, an antigen to the antibody may be used for the capture substance, and a substance (antibody) capable of specifically binding to the antibody may be used for the enzyme-labeled substance.

In the adding step, an anionic surfactant-containing reagent is added to the detection region of the chromatographic test device after the liquid mixture is developed on the chromatographic test device. When an anionic surfactant-containing reagent is present in the detection region, the intensity of the luminescence from the luminescent substrate can be enhanced as described below. The adding step is preferably performed after the capturing step in which the objective substance is captured by the capture substance immobilized on the detection region. When the anionic surfactant-containing reagent is added after the capturing of the objective substance, the anionic surfactant-containing reagent can be placed in the detection region without the anionic surfactant flowing out of the detection region.

The anionic surfactant-containing reagent may be added by any method. For example, the anionic surfactant-containing reagent may be developed on the chromatographic test device so that the reagent can be added to the detection region. Alternatively, the anionic surfactant-containing reagent may be added dropwise to the detection region of the chromatographic test device so that the reagent can be added to the detection region. After the liquid mixture is developed, the chromatographic test device may be immersed in the anionic surfactant-containing reagent so that the reagent can be added to the detection region. Among these methods for adding the anionic surfactant-containing reagent, the method of developing the anionic surfactant-containing reagent on the chromatographic test device is preferred. Particularly when the anionic surfactant-containing reagent further contains a buffer, this method is more preferred, because it can adjust the environment for the luminescence of the labeled substance-objective substance-capture substance and can also clean the chromatographic test device described blow at the same time.

Before the adding step is performed, the process may further include the step of cleaning the chromatographic test device after the capturing step. When the detection region is cleaned, the background can be reduced in the later step of detecting luminescence, so that the objective substance in the sample can be detected with higher sensitivity. The cleaning method to be used may be a known method. For example, a buffer solution containing a buffer agent such as phosphate buffered saline (PBS) or N-tris(hydroxymethyl)methyl-3-aminopropanesulfonic acid (TAPS) may be developed on the chromatographic test device so that the detection region can be cleaned. Alternatively, the chromatographic test device may be immersed in the buffer solution containing a buffer agent so that the detection region can be cleaned.

In the adding step, a reagent containing the anionic surfactant and the buffer may be developed on the chromatographic test device as described above, so that the cleaning step and the adding step can be performed at the same time.

The anionic surfactant may be any known anionic surfactant generally used in the art. Examples of the anionic surfactant include sodium dodecyl sulfate, sodium dodecylbenzenesulfonate, sodium n-decyl sulfate, sodium lauryl sulfoacetate, sodium 1-dodecanesulfonate, p-octylbenzenesulfonic acid, 1-octanesulfonic acid, sodium 1-decanesulfonate, sulfuric acid sodium octane-1-yl, and sodium POE(2) lauryl ether sulfate.

The concentration of the anionic surfactant in the reagent is preferably, but not limited to, from 0.0001 to 0.1% by weight, and it may be appropriately adjusted depending on the type of the anionic surfactant, the luminescent reaction conditions described below, or the like. The intensity of the luminescence from the luminescent substrate can be enhanced at an anionic surfactant concentration of the reagent of 0.0001% by weight or more. The intensity of the luminescence from the luminescent substrate can be sufficiently enhanced at an anionic surfactant concentration of 0.1% by weight or less.

In the reaction step, the luminescent substrate is allowed to react with the enzyme of the enzyme-labeled substance captured in the detection region through the objective substance. If the objective substance is present in the sample, luminescence is generated from the detection region in this step. The enzyme may be allowed to react with the luminescent substance by a method of bringing the enzyme and the luminescent substrate into contact with each other. For example, the reaction step may be performed by adding the luminescent substrate to the detection region with a pipette or the like.

The luminescent substrate to be used may be any appropriate substance capable of causing a luminescent reaction with the enzyme of the enzyme-labeled substance. Examples of the luminescent substrate include luminol, dioxetanes and so on. Dioxetanes (dioxetane compounds) are preferred. Examples of dioxetanes include AMPPD (registered trademark), CSPD (registered trademark), and CDP-Star (registered trademark).

In the detection step, luminescence generated from the detection region in the reaction step is detected so that the objective substance in the sample can be detected. The luminescence may be detected by a method using a known device such as a CCD camera or a fluorescence scanner.
If luminescence is detected in this step, it may be determined that the objective substance is detected. The objective substance in the sample may also be quantified by measuring the intensity of the luminescence detected. The quantification of the objective substance is preferably performed using a calibration curve. A known device may be used in the method of measuring the luminescence intensity. Examples of such a device include Typhoon (manufactured by GE Healthcare Japan) and LAS 4000 (manufactured by FUJIFILM Corporation).

The method for detecting an objective substance according to the invention has been described above. A chromatography test kit according to an embodiment of the invention is described below with reference to the drawings.

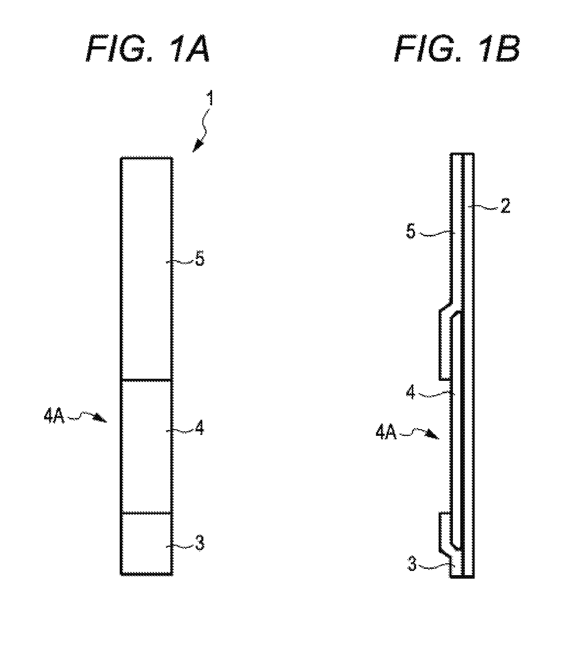

Fig. 1 shows a plan view (part (a)) and a side view (part (b)) of a chromatographic test device 1 (hereinafter referred to as the test device 1) for use in a lateral flow type chromatography method according to a first embodiment of the invention.
As shown in Fig. 1, the test device 1 includes a base member 2 comprising a plastic plate having a sticking surface layer, a sample addition member 3 comprising a nonwoven rayon fabric and provided on the base member 2, a chromatographic membrane carrier 4 comprising a porous nitrocellulose material and provided on the base member 2, and an absorption member 5 comprising a nonwoven cellulose fabric and provided on the base member 2.

The base member 2 is provided to appropriately arrange the sample addition member 3, the chromatographic membrane carrier 4 and the absorption member 5 and may be made of a plastic material or a non-plastic material such as paper or glass.
A sample is added to the sample addition member 3. The sample described above may be used. The sample addition member 3 may be made of a nonwoven rayon fabric or any other material such as cotton, glass fibers, or cellulose fibers.

The chromatographic membrane carrier 4 is placed in contact with the sample addition member 3 and has a detection part 4A. In the detection part 4A, a capture substance capable of specifically binding to an objective substance is immobilized in a line shape.
The absorption member 5 is placed in contact with the chromatographic membrane carrier 4 and provided to absorb the excess part of the sample. The absorption member 5 may be any material capable of quickly absorbing liquid and holding liquid. A nonwoven fabric made of cotton, cellulose, polyethylene, polypropylene, or the like may be used to form the absorption member 5.

Next, a description is given of a method for detecting an objective substance using the chromatographic test device 1 according to the first embodiment.

First, a sample and an enzyme-labeled substance are mixed to form a liquid mixture. In this step, if the sample contains an objective substance, the objective substance and the enzyme-labeled substance bind together to form a complex.
The prepared liquid mixture is added to the sample addition member 3 of the test device 1. The added liquid mixture is developed on the chromatographic membrane carrier 4 from the sample addition member 3. If the sample contains the objective substance, the capture substance immobilized on the detection part 4A captures the complex having the objective substance and the enzyme-labeled substance binding together.
After the added liquid mixture is allowed to stand for about 1 minute, an anionic surfactant-containing cleaning liquid is added to the sample addition member 3. The added cleaning liquid is developed on the chromatographic membrane carrier 4 and comes into contact with the detection part 4A.
After the added cleaning liquid is allowed to stand for about 1 minute, a luminescent substrate is added to the detection part 4A. In this step, if the capture substance in the detection part 4A captures the complex, the luminescent substrate reacts with the enzyme of the enzyme-labeled substance in the complex to generate luminescence.
After the luminescent substrate is added, luminescence is detected using a luminescence detector (Typhoon manufactured by GE Healthcare Japan). The objective substance can be detected by detecting the luminescence. In addition, the intensity of the luminescence detected may be measured so that the objective substance can be quantified using a calibration curve.

While the structure in the embodiment described above has the sample addition member 3 for receiving a sample, the sample addition member 3 may be omitted from the structure. In such a case, the sample may be added directly to the chromatographic membrane carrier 4 and developed thereon.
In the embodiment described above, the luminescent substrate is added to the detection part 4A. Alternatively, the luminescent substrate may be added to the sample addition member 3 and developed or may be added directly to the chromatographic membrane carrier 4 and developed thereon.

In the embodiment described above, a reagent containing an anionic surfactant and a buffer as described above may be used as the anionic surfactant-containing cleaning liquid.

The chromatographic membrane carrier 4 may have a single detection part or two or more detection parts. The chromatographic membrane carrier 4 may also have a reference part. For example, an ALP-labeled anti-HBs antibody may be added to the sample, and an anti-mouse IgG antibody capable of binding to the ALP-labeled anti-HBs antibody may be immobilized on the reference part of the chromatographic membrane carrier 4 to be used.

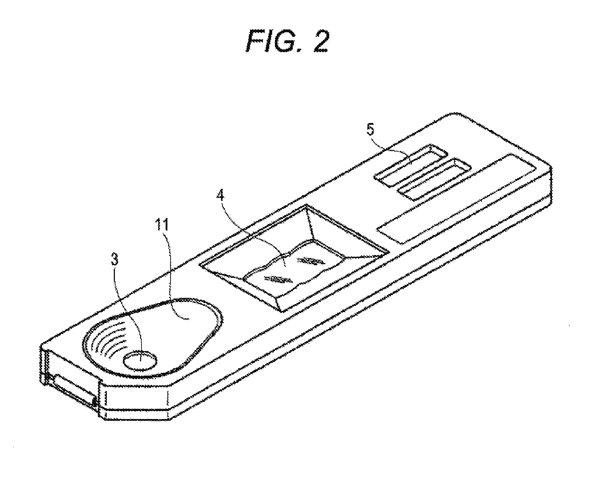

In the embodiment described above, the test device 1 may be housed in a case 10 having openings at locations corresponding to the sample addition member 3, the chromatographic membrane carrier 4 and the absorption member 5. Fig. 2 shows an example of the test device 1 having such a structure.
When the test device 1 is housed in the case 10 as shown in Fig. 2, the sample can be prevented from leaking out of each member of the test device 1, so that the objective substance contained in the sample can be detected in a sanitary manner. In addition, the liquid component of the sample can be easily evaporated from the openings formed in the case 10, so that the development of the sample can be facilitated.

In the structure shown in Fig. 2, the opening 11 of the case 10 at the location corresponding to the sample addition member 3 is formed so that the opening area becomes smaller toward the inner end. In such a structure, a constant amount of the sample added to the sample addition member 3 is stored in the opening 11. Therefore, even if the sample is added in an excess amount, the sample can be successively developed on the test device 1, so that the test can be performed without periodical addition of a small amount of the sample.

While the lateral flow type chromatographic test device 1 has been described above, the chromatography test kit according to an embodiment of the invention may include a flow through type chromatographic test device. Such a flow through type chromatographic test device is described below with reference to the drawings.

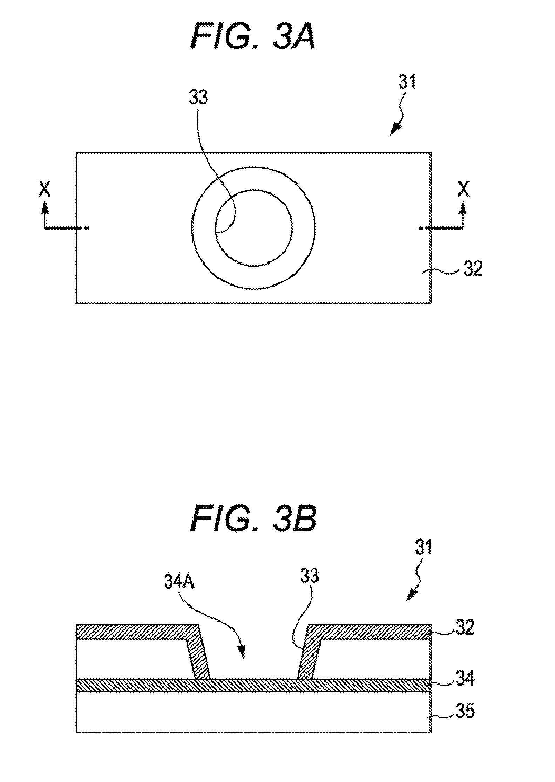

Fig. 3 shows a plan view (part (a)) and an X-X cross-sectional view (part (b)) of a chromatographic test device 31 (hereinafter referred to as the test device 31) for use in a flow through type chromatography method according to a second embodiment of the invention.

The test device 31 includes an absorption member 35, a chromatographic membrane carrier 34 and a cover member 32 which are laminated in this order from the lower layer.
The cover member 32 has an opening 33, and the chromatographic membrane carrier 34 placed under it has a detection part 34A which is exposed through the opening 33.

The absorption member 35 is placed in contact with the chromatographic membrane carrier 34 and is provided to absorb the excess part of the sample. The absorption member 35 may be any material capable of quickly absorbing liquid and holding liquid. A nonwoven fabric made of cotton, cellulose, polyethylene, polypropylene, or the like may be used to form the absorption member 35.
The chromatographic membrane carrier 34 is placed in contact with the cover member 32 and has a detection part 34A. In the detection part 34A, a capture substance capable of specifically binding to an objective substance is immobilized in a line shape.

Next, a description is given of a method for detecting an objective substance using the flow through type chromatographic test device 31 according to the second embodiment.
First, a sample and an enzyme-labeled substance are mixed to form a liquid mixture. In this step, if the sample contains an objective substance, the objective substance and the enzyme-labeled substance bind together to form a complex. The prepared liquid mixture is added to the opening 33 of the test device 31 and allowed to stand for about 20 minutes in this state. In this step, the liquid mixture passes through the chromatographic membrane carrier 34 and is absorbed by the absorption membrane 35 placed under it. If the sample contains the objective substance, the capture substance immobilized on the detection part 34A of the chromatographic membrane carrier 34 captures the complex of the objective substance and the enzyme-labeled substance.
An anionic surfactant-containing cleaning liquid is then added to the opening 33. The added cleaning liquid comes into contact with the detection part 34A. After the cleaning liquid is added, a luminescent substrate is added to the detection part 34A. In this step, if the capture substance in the detection part 34A captures the complex, the luminescent substrate reacts with the enzyme of the enzyme-labeled substance in the complex to generate luminescence.
After the luminescent substrate is added, luminescence is detected using a luminescence detector (Typhoon manufactured by GE Healthcare Japan). The objective substance can be detected by detecting the luminescence. In addition, the intensity of the luminescence detected may be measured so that the objective substance can be quantified using a calibration curve.

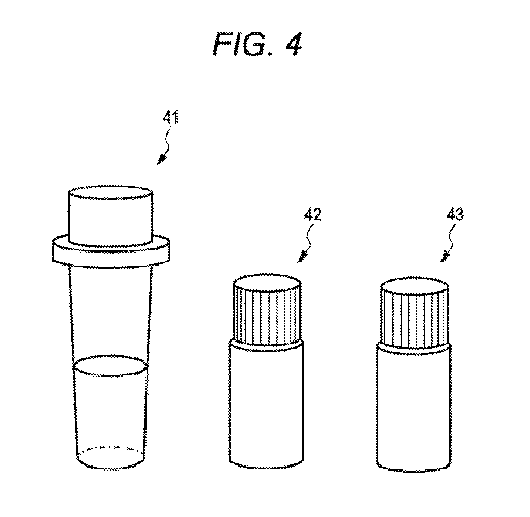

Referring to Fig. 4, the reagent kit of the invention may include a first reagent vessel 41 containing an enzyme-labeled substance, a second reagent vessel 42 containing an anionic surfactant-containing reagent, a third reagent vessel 43 containing a luminescent substrate, and the test device shown in Fig. 1, 2 or 3.

The enzyme-labeled substance contained in the first reagent vessel 41 may form a solution in a buffer or may be in the form of a solid, which is obtained by freeze drying or the like and to which liquid such as water is added before use. In view of easy handling of the reagent kit, the enzyme-labeled substance is preferably in the form of a solution in a buffer.
The first reagent vessel 41 may further contain a pretreatment reagent. In this case, even when the sample needs a pretreatment, the sample can be subjected to a pretreatment and labeling with an enzyme at the same time. Any pretreatment reagent with which the objective substance can be extracted from the sample may be used. For example, a surfactant-containing buffer may be used.

The second reagent vessel 42 may contain the anionic surfactant-containing reagent described above for the detection method.
The luminescent substrate contained in the third reagent vessel 43 may be deactivated by temperature or light. Therefore, the luminescent substrate is preferably stored in a light-tight vessel at low temperature (2 to 8°C).

If desired, the reagent kit described above may further include a further reagent vessel containing any other reagent. If desired, the reagent kit may further include one or more buffers for use in diluting one or more of the reagents, instructions, a vessel for use in a reaction, or the like.

The invention is more specifically described by the examples below, which are not intended to limit the scope of the invention.

### EXAMPLES

Example 1
Detection Sensitivity When Adding Anionic Surfactant after Sample Development
The sensitivity of the detection of luminescence was examined when an anionic surfactant was added after a sample was developed.

### (1) Preparation of Test Device

A lateral flow type chromatographic test device as shown in Fig. 1 was prepared using an HBs antibody.
First, an HBs antibody was diluted to a concentration of 2 mg/mL with a phosphate buffer solution (pH 7.0), and the resulting HBs antibody solution was applied with a width of 1.0 mm to the detection part of a chromatographic membrane carrier made of a nitrocellulose membrane using an antibody coater (manufactured by BioDot, Inc.) and dried at 50°C for 30 minutes. After the drying, the chromatographic membrane carrier was immersed in a BSA-containing phosphate buffer solution (pH 7.0) so that the HBs antibody was immobilized on the detection part of the chromatographic membrane carrier. Thereafter, the membrane carrier was washed with a phosphate buffer solution (pH 7.0) and dried at 40°C for 120 minutes, so that a chromatographic membrane carrier having the HBs antibody bonded thereto was obtained.

### (2) Preparation of Alkaline Phosphatase-Labeled Anti-HBs Antibody Solution

Twenty µL of an ALP-labeled anti-HBs antibody solution was prepared. It has the following composition.

ALP-labeled anti-HBs antibody solution

| | |
|---|---|
| MES | 4.25 g/L (pH 6.5) |
| MgCl₂·6H₂O | 0.04 g/L |
| Sodium chloride | 1.75 g/L |
| BSA | 0.20 g/L |
| Sodium caseinate | 1 g/L |
| ALP-labeled antibody mixture | 3 mL/L |

In the preparation of the ALP-labeled antibody mixture, EMCS (manufactured by DOJINDO LABORATORIES) was used to label the HBs antibody with ALP. The resulting ALP-labeled anti-HBs antibody was dissolved at 0.3 U/mL in an MES buffer solution (pH 6.5) to form the ALP-labeled antibody mixture.

### (3) Development of Sample

An HBs antigen (at a concentration of 0.1 IU/mL) was used as an objective substance. Twenty µl of the ALP-labeled anti-HBs antibody solution prepared as described above and 70 µL (0.1 IU/mL) of the HBs antigen were mixed to form a liquid mixture containing a complex of the HBs antigen and ALP. The prepared liquid mixture was then developed on the chromatographic test device.

### (4) Preparation of Anionic Surfactant-Containing Cleaning Liquid

An anionic surfactant-containing cleaning liquid having the composition shown below was prepared. Cleaning Liquid Composition
50 mM TAPS (pH 8.5)
3 mM MgCl₂·6H₂O
Anionic surfactant (0.005% sodium dodecylbenzenesulfonate (DBS)) The cleaning was performed by developing 50 µL of the cleaning liquid twice on the chromatographic test device.

### (5) Detection of Luminescence

After the cleaning liquid was developed, 50 µL of a luminescent substrate was added to the detection part of the chromatographic membrane carrier. The luminescent substrate used was CDP-Star (registered trademark) (manufactured by Applied Biosystems).

After the addition, Typhoon (manufactured by GE Healthcare Japan) was used to detect luminescence. The detection method was performed according to the instructions for use of Typhoon.

In the step (3), the HBs antigen concentration was changed to 0 IU/mL, 0.0031 IU/mL, 0.0063 IU/mL, 0.0125 IU/mL, 0.025 IU/mL, 0.05 IU/mL, 0.5 IU/mL, 1 IU/mL, and 10 IU/mL, and luminescence was also detected in the same way.

For comparison, an anionic surfactant-free cleaning liquid was used in the step (4), and luminescence was detected in the same way.

Comparative Example 1 Detection Sensitivity When Adding Anionic Surfactant to Test Sample
The sensitivity of the detection of luminescence was examined using the process of Example 1, except that the anionic surfactant was not added to the cleaning liquid in the step (4) of Example 1 but added to the liquid mixture in the step (3) of Example 1.

### (1) Preparation of Test Device

The test device was prepared as in the step (1) of Example 1.

### (2) Preparation of Alkaline Phosphatase-Labeled Anti-HBs Antibody Solution

The alkaline phosphate-labeled anti-HBs antibody solution was prepared as in the step (2) of Example 1.

### (3) Development of Objective Substance

The objective substance used was an HBs antigen (at a concentration of 0.1 IU/mL). Twenty µL of the ALP-labeled anti-HBs antibody solution prepared in the step (2) and 70 µL of the HBs antigen were mixed to form a liquid mixture containing a complex of the HBs antigen and ALP.

DBS, an anionic surfactant, was added at a concentration of 0.005% to the liquid mixture. The DBS-containing liquid mixture was then developed on the chromatographic test device.

### (4) Preparation of Cleaning Liquid

After the DBS-containing liquid mixture was developed, a cleaning liquid having the composition shown below was prepared for the cleaning of the chromatographic test device.
Cleaning Liquid Composition
50 mM TAPS (pH 8.5)
3 mM MgCl₂·6H₂O Fifty µL of the cleaning liquid was developed twice on the chromatographic test device.

### (5) Detection of Luminescence

The process was performed as in the step (5) of Example 1.

The concentration of DBS added in the step (3) was changed to 0%, 0.01%, 0.025%, and 0.05%, and the detection sensitivity was also examined in the same way. In addition, the HBs antigen concentration was changed to 0.5 IU/mL, and the detection sensitivity was also examined in the same way.

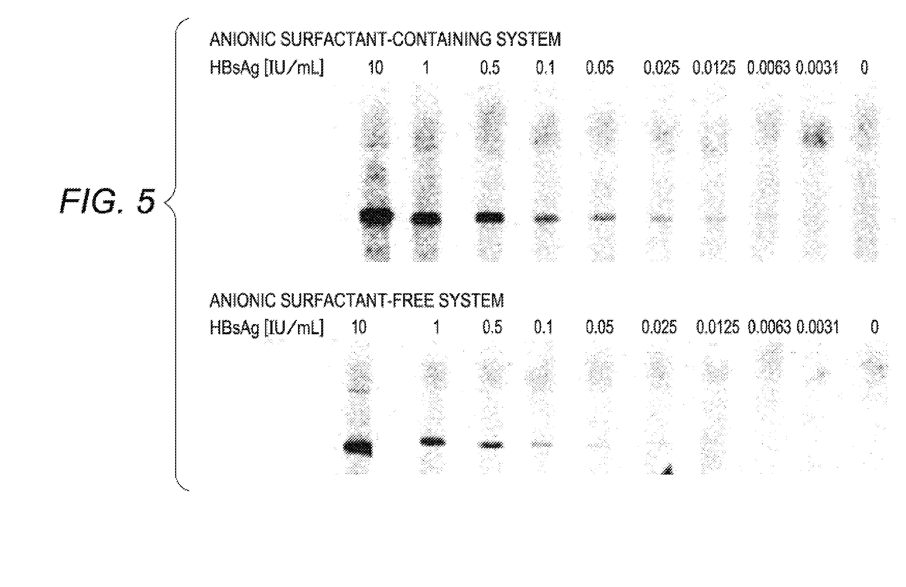

Result Fig. 5 is a photograph showing the result of the detection of luminescence in Example 1. Fig. 5 shows that in the DBS-containing system, the objective substance (HBs antigen) was detectable at a concentration of as low as 0.0063 IU/mL, whereas in the DBS-free system, the objective substance was detectable at a concentration of 0.025 IU/mL or more. It has therefore been suggested that the cleaning with the anionic surfactant-containing liquid after the development of the sample leads to an increase in luminescence intensity and an increase in the sensitivity of the detection of the objective substance.

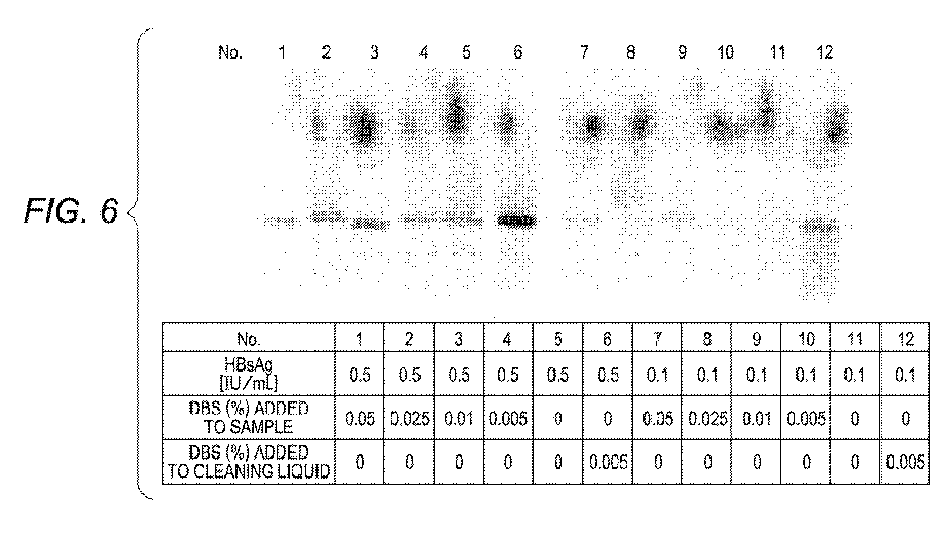

Fig. 6 is a photograph showing the result of the detection of luminescence in Comparative Example 1. Fig. 6 shows that no difference in detection sensitivity was observed between Sample Nos. 1-4 and 7-10 prepared with DBS added to the liquid mixture and Sample No. 5 or 11 prepared without the addition of DBS. This has suggested that neither the luminescence intensity nor the detection sensitivity is changed by the addition of the anionic surfactant to the sample to be developed.
Thus, it has been suggested that when the sample containing a complex of the objective substance and the enzyme-labeled substance is developed and then washed with the anionic surfactant-containing liquid, the luminescence intensity and the sensitivity of the detection of the objective substance become higher.

Example 2 Sensitivity of Detection Performed with Various Surfactants
The detection sensitivity was examined using various surfactants.
The surfactants used were nonionic surfactants including polyoxyethylene lauryl ether (NIKKOL BL-25 manufactured by Nikko Chemicals co., ltd.), polyoxyethylene nonylphenyl ether (NP-40 manufactured by NACALAI TESQUE, INC.), polyoxyethylene octylphenyl ether (NIKKOL HS-240 manufactured by Nikko Chemicals co., ltd.), polyoxyethylene-polyoxypropylene copolymer (UNILUB 70DP-950 manufactured by NOF CORPORATION), and dodecyl maltoside (DM manufactured by DOJINDO LABORATORIES); a cationic surfactant including myristyl trimethyl ammonium bromide (MTAB manufactured by Wako Pure Chemical Industries, Ltd.); an anionic surfactant including sodium dodecyl sulfate (SDS manufactured by NACALAI TESQUE, INC.); and an ampholytic surfactant including a cholic acid derivative (CHAPS manufactured by DOJINDO LABORATORIES).
The measurement was performed by the same method as in Example 1, except that each of the surfactants listed above was used in the step (4) of preparing the cleaning liquid.

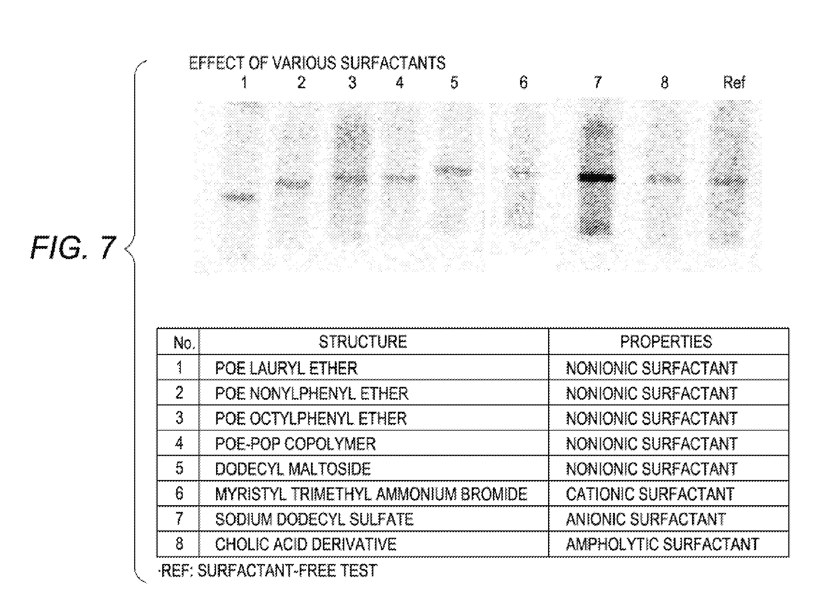

Result Fig. 7 is a photograph showing the result of the detection of luminescence when various nonionic, cationic, anionic, and ampholytic surfactants were used. Fig. 7 indicates that only when SDS, an anionic surfactant, is added, the luminescence intensity becomes higher. Thus, it has been suggested that only when an anionic surfactant is added, the luminescence intensity and the sensitivity of the detection of the objective substance in the sample may become higher.

Example 3
Sensitivity of Detection Performed with Various Anionic Surfactants
The detection sensitivity was examined using various anionic surfactants. The measurement was performed by the same method as in Example 1, except that each of the anionic surfactants shown below was used in the step (4) of preparing the cleaning liquid. Anionic Surfactants Used
1. Sodium lauryl sulfoacetate (LSA-F) 0.01%
2. Sodium 1-dodecanesulfonate 0.01%
3. p-octylbenzenesulfonic acid 0.01%
4. 1-octanesulfonic acid 0.01%
5. 1-decanesulfonic acid 0.01%
6. Sodium octyl sulfate 0.01%
7. Sodium polyoxyethylene lauryl ether sulfonate (SBL-2N-27) 0.01%
8. Sodium n-decyl sulfate 0.01%
9. Sodium dodecyl sulfate 0.01%
10. Sodium dodecylbenzenesulfonate 0.01%

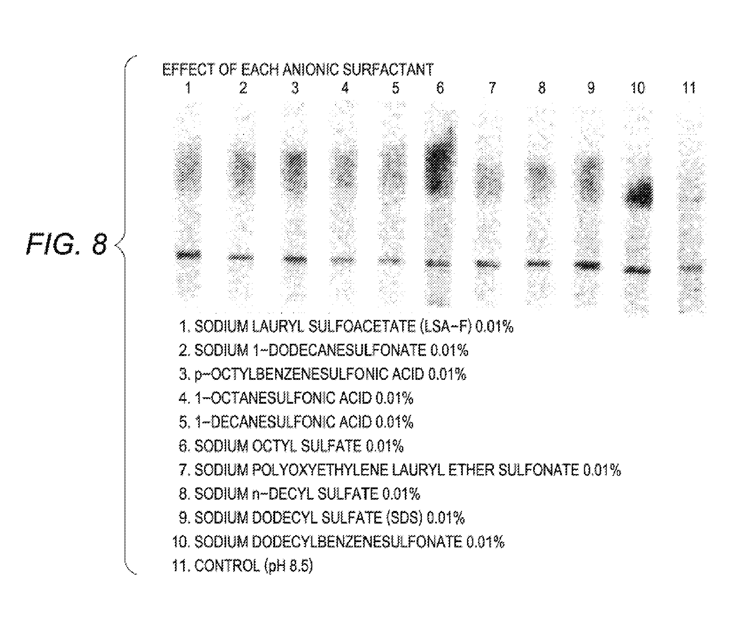

Result Fig. 8 is a photograph showing the result of the detection of luminescence when each of the anionic surfactants listed above was added. Fig. 8 indicates that the use of any of the anionic surfactants makes it possible to increase the luminescence intensity. Thus, it has been suggested that the use of an anionic surfactant makes it possible to increase the luminescence intensity and the sensitivity of the detection of the objective substance in the sample.

Example 4 Sensitivity of Detection Performed at Different Anionic Surfactant Concentrations The detection sensitivity was examined using various anionic surfactant concentrations. The detection was performed by the same method as in Example 1, except that the anionic surfactant was used at concentrations of 0.001%, 0.005%, 0.01%, and 0.05% in in the step (4) of Example 1.

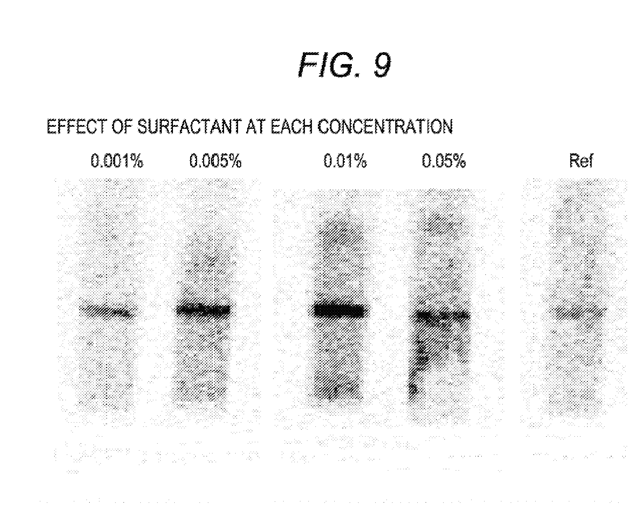

Result Fig. 9 is a photograph showing the result of the detection of luminescence when various anionic surfactant concentrations were used. Fig. 9 shows that as the anionic surfactant concentration increases from 0.001% to 0.01%, the luminescence intensity increases and that the luminescence intensity is higher at 0.01% than at 0.05%. Thus, it has been suggested that a 0.01% addition of an anionic surfactant can sufficiently increase the luminescence intensity and increase the sensitivity of the detection of an objective substance in a sample.

## Claims

1. A method for detecting an objective substance in a sample using a chromatographic test device having a detection region on which a capture substance capable of binding to the objective substance is immobilized, comprising:
mixing the sample and an enzyme-labeled substance capable of binding to the objective substance;
trapping the objective substance binding to the enzyme-labeled substance by the capture substance in the detection region, by adding the liquid mixture to the chromatographic test device;
adding an anionic surfactant-containing reagent to the detection region of the chromatographic test device in which the objective substance is captured;
reacting a luminescent substrate with the enzyme of the enzyme-labeled substance binding to the capture substance through the objective substance in the detection region; and
detecting the objective substance in the sample by detecting luminescence generated from the detection region by the reaction of the luminescent substrate with the enzyme.

2. The method of claim 1, wherein the anionic surfactant is selected from sodium dodecyl sulfate, sodium dodecylbenzenesulfonate, sodium n-decyl sulfate, sodium lauryl sulfoacetate, sodium 1-dodecanesulfonate, p-octylbenzenesulfonic acid, 1-octanesulfonic acid, sodium 1-decanesulfonate, sulfuric acid sodium octane-1-yl, and sodium POE(2) lauryl ether sulfate.

3. The method of claim 1 or claim 2, wherein the addition of the anionic surfactant-containing reagent to the detection region of the chromatographic test device is performed by developing the anionic surfactant-containing reagent on the chromatographic test device.

4. The method of any one of claims 1 to 3, wherein the anionic surfactant-containing reagent further contains a buffer; and
the anionic surfactant-containing reagent contains the anionic surfactant at a concentration of 0.0001 to 0.1% by weight.

5. The method of any one of claims 1 to 4, wherein the luminescent substrate is a dioxetane; and
the enzyme of the enzyme-labeled substance is alkaline phosphatase.

6. The method of any one of claims 1 to 5, wherein the capture substance is an antibody, and the enzyme-labeled substance is an enzyme-labeled antibody.

7. The method of any one of claims 1 to 6, wherein the detection of the objective substance comprises quantifying the objective substance in the sample by measuring the intensity of the luminescence generated from the detection region.

8. The method of any one of claims 1 to 7, wherein the chromatographic test device is a lateral flow type chromatographic test device or a flow through type chromatographic test device.

9. A chromatography test kit for detecting an objective substance in a sample, comprising:
an enzyme-labeled substance capable of binding to the objective substance;
a chromatographic test device having a detection region on which a capture substance capable of binding to the objective substance is immobilized;
an anionic surfactant-containing reagent; and
a luminescent substrate for the enzyme-labeled substance, the substrate being capable of acting on the enzyme-labeled substance and causing a luminescent reaction.

10. The kit of claim 9, wherein the anionic surfactant is selected from sodium dodecyl sulfate, sodium dodecylbenzenesulfonate, sodium n-decyl sulfate, sodium lauryl sulfoacetate, sodium 1-dodecanesulfonate, p-octylbenzenesulfonic acid, 1-octanesulfonic acid, sodium 1-decanesulfonate, sulfuric acid sodium octane-1-yl, and sodium POE(2) lauryl ether sulfate.

11. The kit of claim 9 or claim 10, wherein the anionic surfactant-containing reagent further contains a buffer; and
the anionic surfactant-containing reagent contains the anionic surfactant at a concentration of 0.0001 to 0.1% by weight.

12. The kit of any one of claims 9 to 11, wherein the luminescent substrate is a dioxetane.

13. The kit of any one of claims 9 to 12, wherein the enzyme of the enzyme-labeled substance is alkaline phosphatase.

14. The kit of any one of claims 9 to 13, wherein the capture substance is an antibody, and the enzyme-labeled substance is an enzyme-labeled antibody.

15. The kit of any one of claims 9 to 14, wherein the chromatographic test device is a lateral flow type chromatographic test device or a flow through type chromatographic test device.
